# EUROPÄISCHE PATENTSCHRIFT

(11) **EP 1 368 301 B1**
(45) Veröffentlichungstag und Bekanntmachung des Hinweises auf die Patenterteilung: **31.10.2007**
(21) Anmeldenummer: 02716805.3
(22) Anmeldetag: 22.02.2002
(51) Int. Cl.: C07C 229/38, A61K 31/195, A61P 9/00

(54) **SEITENKETTENHALOGENIERTE AMINODICARBONSAUREDERIVATE ALS ARZNEIMITTEL ZUR BEHANDLUNG VON HERZ-KREISLAUF-ERKRANKUNGEN**
SIDE-CHAIN HALOGENATED AMINO CARBOXYLIC ACID DERIVATIVES AS MEDICAMENTS FOR TREATING CARDIOVASCULAR DISEASES
DERIVES D'ACIDE AMINODICARBOXYLIQUE A HALOGENATION DE CHAINE LATERALE SERVANT D'AGENTS PHARMACEUTIQUES POUR LE TRAITEMENT DE MALADIES CARDIO-VASCULAIRES

(30) Priorität: 01.03.2001 DE 10109861
(43) Veröffentlichungstag der Anmeldung: 10.12.2003
(73) Patentinhaber: Bayer HealthCare AG, 51368 Leverkusen (DE)
(72) Erfinder: HÄRTER, Michael, 51375 Leverkusen (DE); HAHN, Michael, 40764 Langenfeld (DE); HIRTH-DIETRICH, Claudia, 42115 Wuppertal (DE); KNORR, Andreas, 40699 Erkrath (DE); STAHL, Elke, 51467 Bergisch Gladbach (DE); STASCH, Johannes, Peter, 42651 Solingen (DE); WUNDER, Frank, 42117 Wuppertal (DE)
(86) Internationale Anmeldenummer: PCT/EP2002/001895
(87) Internationale Veröffentlichungsnummer: WO 2002/070461

(56) Entgegenhaltungen:
- EP-A- 0 341 551
- WO-A-01/19780

## Beschreibung

Die vorliegende Erfindung betrifft neue seitenkettenhalogenierte Aminocarbonsäurederivate, welche die lösliche Guanylatcyclase auch über einen neuartigen, ohne Beteiligung der Häm-Gruppe des Enzyms verlaufenden Wirkmechanismus stimulieren, ihre Herstellung und ihre Verwendung als Arzneimittel, insbesondere als Arzneimittel zur Behandlung von Herz-Kreislauf Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriposphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch CO ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NOunabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisenzentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol (YC-1, Wu et al., Blood 84 (1994), 4226; Mülsch et al., Br.J.Pharmacol. 120 (1997), 681), Fettsäuren (Goldberg et al, J. Biol. Chem. 252 (1977), 1279), Diphenyliodonium-hexafluorophosphat (Pettibone et al., Eur. J. Pharmacol. 116 (1985), 307), Isoliquiritigenin (Yu et al., Brit. J. Pharmacol. 114 (1995), 1587), sowie verschiedene substituierte Pyrazolderivate (WO 98/16223, WO 98/16507 und WO 98/23619).

Die vorstehend beschriebenen Stimulatoren der löslichen Guanylatcyclase stimulieren das Enzym entweder direkt über die Häm-Gruppe (Kohlenmonoxid, Stickstoffmonoxid oder Diphenyliodoniumhexafluorophosphat) durch Interaktion mit dem Eisenzentrum der Häm-Gruppe und eine sich daraus ergebende, zur Erhöhung der Enzymaktivität führende Konformationsänderung (Gerzer et al., FEBS Lett. 132(1981), 71), oder über einen Häm-abhängigen Mechanismus, der unabhängig von NO ist, aber zu einer Potenzierung der stimulierenden Wirkung von NO oder CO führt (z.B. YC-1, Hoenicka et al., J. Mol. Med. (1999) 14; oder die in der WO 98/16223, WO 98/16507 und WO 98/23619 beschriebenen Pyrazolderivate).

Die in der Literatur behauptete stimulierende Wirkung von Isoliquiritigenin und von Fettsäuren, wie z. B. Arachidonsäure, Prostaglandinendoperoxide und Fettsäurehydroperoxide auf die lösliche Guanylatcyclase konnte nicht bestätigt werden (vgl. z.B. Hoenicka et al., J. Mol. Med. 77 (1999), 14).

Entfernt man von der löslichen Guanylatcyclase die Häm-Gruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten bekannten Stimulatoren stimulierbar.

Es wurde eine Stimulation von Häm-freier löslicher Guanylatcyclase durch Protoporphyrin IX beschrieben (Ignarro et al., Adv. Pharmacol. 26 (1994), 35). Allerdings kann Protoporphyrin IX als Mimik für das NO-Häm-Addukt angesehen werden, weshalb die Zugabe von Protoporphyrin IX zur löslichen Guanylatcyclase zur Bildung einer der durch NO stimulierten Häm-haltigen löslichen Guanylatcyclase entsprechenden Struktur des Enzyms führen dürfte. Dies wird auch durch die Tatsache belegt, dass die stimulierende Wirkung von Protoporphyrin IX durch den vorstehend beschriebenen NO-unabhängigen, aber Häm-abhängigen Stimulator YC-1 erhöht wird (Mülsch et al., Naunyn Schmiedebergs Arch. Pharmacol. 355, R47).

Im Gegensatz zu den vorstehend beschriebenen, aus dem Stand der Technik als Stimulatoren der löslichen Guanylatcyclase bekannten Verbindungen sind die erfindungsgemäßen Verbindungen in der Lage, sowohl die Häm-haltige als auch die Häm-freie Form der löslichen Guanylatcyclase zu stimulieren. Die Stimulierung des Enzyms verläuft bei diesen neuen Stimulatoren also über einen Häm-unabhängigen Weg, was auch dadurch belegt wird, dass die neuen Stimulatoren am Häm-haltigen Enzym einerseits keine synergistische Wirkung mit NO zeigen und andererseits sich die Wirkung dieser neuartigen Stimulatoren nicht durch den Häm-abhängigen Inhibitor der löslichen Guanylatcyclase, 1*H*-1,2,4-Oxadiazol-(4,3-a)-chinoxalin-1-on (ODQ), blockieren lässt.

EP-A-0 341 551 offenbart Alkensäurederivate als Medikamenten zur Behandlung von Krankheiten des Kreislaufsystems.

Dies stellt einen neuen Therapieansatz zur Behandlung von Herz-Kreislauf-erkrankungen und anderen über eine Beeinflussung des cGMP-Signalweges in Organismen therapierbaren Erkrankungen dar.

In der EP-A-0 345 068 ist unter anderem die Aminoalkancarbonsäure (1) als Zwischenprodukt bei der Synthese von GABA-Antagonisten beschrieben:

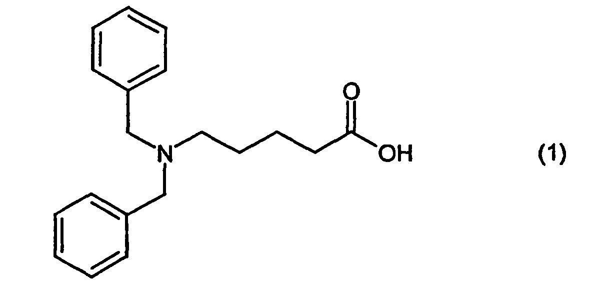

In der WO 93/00359 ist die Aminoalkancarbonsäure (2) als Intermediat in der PeptidSynthese sowie dessen Verwendung als Wirkstoff zur Behandlung von Erkrankungen des zentralen Nervensystems beschrieben:

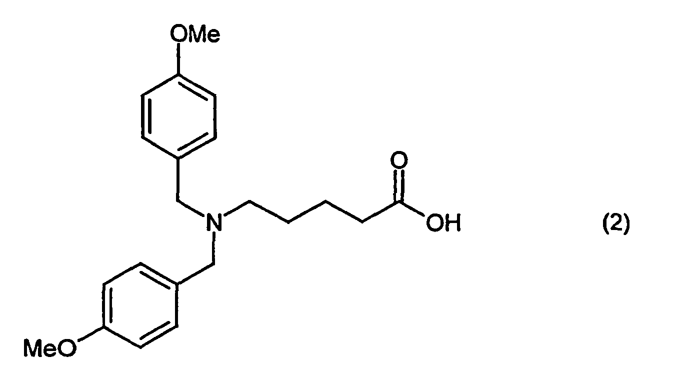

In keiner dieser beiden Schriften ist jedoch beschrieben, dass derartige Aminoalkancarbonsäuren einen von der im Enzym befindlichen Häm-Gruppe unabhängigen stimulierenden Effekt auf die lösliche Guanylatcyclase ausüben können.

Den erfindungsgemäßen Verbindungen strukturell ähnliche Substanzen sind darüber hinaus aus WO 01/19776, WO 01/19355, WO 01/19780 und WO 01/19778 bekannt.

Die vorliegende Erfindung betrifft Verbindungen der allgemeinen Formel (I) 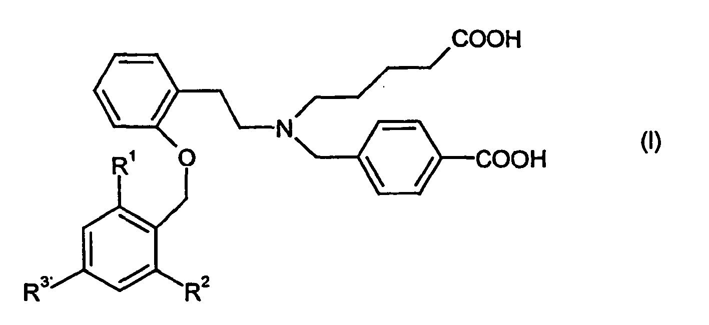 worin
- R¹: Halogen bedeutet;
- R²: H oder Halogen bedeutet;
- R³: C₃₋₈-Cycloalkyl, C₃₋₈-Cycloalkenyl oder Phenyl bedeutet, wobei der Phenylrest zusätzlich einen Substituenten aus der Gruppe, bestehend aus Halogen, C₁₋₆-Alkyl, C₁₋₆-Alkoxy, CF₃, tragen kann;
sowie Salze, Isomere und Hydrate davon.

Gemäß einer bevorzugten Ausführungsform betrifft die vorliegende Erfindung Verbindungen der Formel (I), worin
- R¹: F oder Cl bedeutet;
- R²: H bedeutet;
- R³: C₃₋₈-Cycloalkyl, C₃₋₈-Cycloalkenyl oder Phenyl bedeutet, wobei der Phenylrest zusätzlich einen Substituenten aus der Gruppe, bestehend aus F, Cl, Br, Methyl, Ethyl, n-Propyl, i-Propyl, n-Butyl, i-Butyl, t-Butyl, Methoxy, Ethoxy, n-Propoxy, i-Propoxy, n-Butyloxy, i-Butyloxy, t-Butyloxy, CF₃, tragen kann;
sowie Salze, Isomere und Hydrate davon.

Gemäß einer besonders bevorzugten Ausführungsform betrifft die vorliegende Erfindung Verbindungen der Formel (I), worin
- R¹: F oder Cl bedeutet;
- R²: H bedeutet;
- R³: Cyclohexyl, 1-Cyclohexenyl oder Phenyl bedeutet, wobei der Phenylrest zusätzlich einen Substituenten aus der Gruppe, bestehend aus F, Cl, t-Butyl,OMe, CF₃, tragen kann;
sowie Salze, Isomere und Hydrate davon.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, p-Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren Die Erfindung betrifft sowohl die Enantiomeren oder Diastereomeren als auch deren jeweilige Mischungen. Die Racemformen lassen sich ebenso wie die Diastereomeren in bekannter Weise, beispielsweise durch Racematspaltung oder chromatographische Trennung, in die stereoisomer einheitlichen Bestandteile trennen. In den erfindungsgemäßen Verbindungen vorhandene Doppelbindungen können in der cis- oder trans- Konfiguration (Z- oder E-Form) vorliegen.

Im Rahmen der vorliegenden Erfindung haben die Substituenten soweit nicht anders angegeben im allgemeinen die folgende Bedeutung:
Alkyl steht im allgemeinen für einen geradkettigen oder verzweigten Kohlenwasserstoffrest mit 1 bis 20 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl, Nonyl, Decyl, Dodeyl, Eicosyl genannt.
Alkoxy steht im allgemeinen für einen über einen Sauerstoffatom gebundenen geradkettigen oder verzweigten Kohlenwasserstoffrest mit 1 bis 14 Kohlenstoffatomen. Beispielsweise seien Methoxy, Ethoxy, Propoxy, Isopropoxy, Butoxy, Isobutoxy, Pentoxy Isopentoxy, Hexoxy, Isohexoxy, Heptoxy, Isoheptoxy, Octoxy oder Isooctoxy genannt. Die Begriffe "Alkoxy" und "Alkyloxy" werden synonym verwendet.
Cycloalkyl steht im allgemeinen für einen cyclischen Kohlenwasserstoffrest mit 3 bis 8 Kohlenstoffatomen. Bevorzugt sind Cyclopropyl, Cyclopentyl und Cyclohexyl. Beispielsweise seien Cyclopentyl, Cyclohexyl, Cycloheptyl und Cyclooctyl genannt.
Cycloalkenyl steht im allgemeinen für einen cyclischen Kohlenwasserstoffrest mit 3 bis 8 Kohlenstoffatomen, der zusätzlich eine Doppelbindung im Ring aufweist. Bevorzugt sind Cyclopropenyl, Cyclopentenyl und Cyclohexenyl. Beispielsweise seien Cyclopentenyl, Cyclohexenyl, Cycloheptenyl und Cyclooctenyl genannt.
Halogen steht im Rahmen der Erfindung für Fluor, Chlor, Brom und Iod.

Die vorliegende Erfindung betrifft weiterhin ein Verfahren zur Herstellung der Verbindungen der Formel (I), dadurch gekennzeichnet, dass man

Verbindungen der Formel (II) 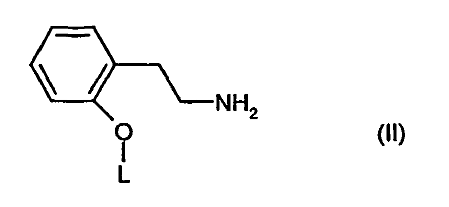 worin
- L: für Methyl steht,
mit einem 4-Formylbenzoesäure-C₁₋₆-alkylester in einem organischen Lösungsmittel gegebenenfalls unter Erhitzen und gleichzeitiger oder anschließender Zugabe eines Reduktionsmittels zu Verbindungen der Formel (III) umsetzt, 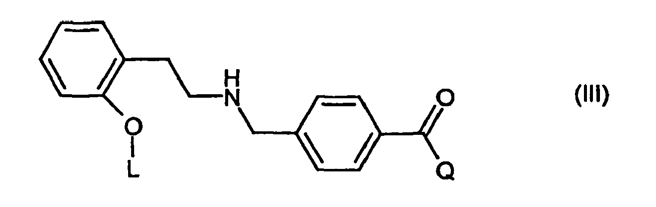 worin
- L: die vorstehend angegebene Bedeutung hat und Q für einen C₁₋₆-Alkylrest steht,
anschließend -unter vorheriger Etherspaltung zur freien Hydroxylgruppe - mit einem ω-Halogenvaleriansäure-C₁₋₆-alkylester in einem organischen Lösungsmittel in Gegenwart einer Base unter Erhitzen zu Verbindungen der Formel (IV) umsetzt, 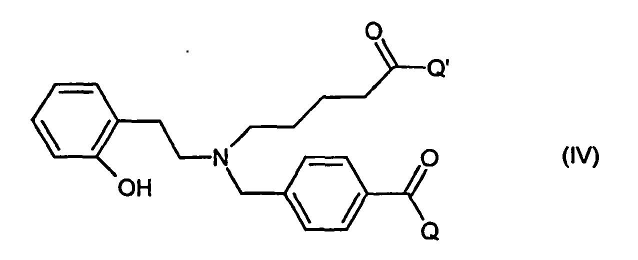 worin
- Q: die vorstehend angegebene Bedeutung hat und Q' für einen C₁₋₆-Alkylrest steht,
anschließend mit einer Verbindung der Formel IV-A in einem organischen Lösungsmittel in Gegenwart einer Base unter Erhitzen 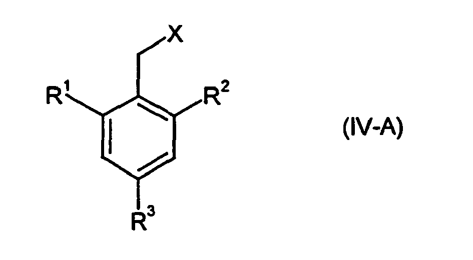 wobei
- R¹, R² und R³: die vorstehend angegebenen Bedeutung haben und X für Halogen steht,
zu Verbindungen der Formel (V) umsetzt, 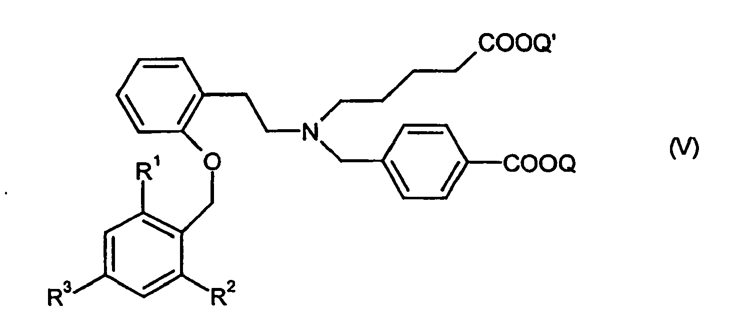 und anschließend die Verbindungen der Formel (V) unter alkalischen Bedingungen zu den Verbindungen der Formel (I) hydrolysiert.

Die für die erfindungsgemäßen Verfahren bevorzugten Basen umfassen herkömmlicherweise für basische Reaktionen eingesetzte basische Verbindungen. Vorzugsweise können Alkalimetallhydride wie beispielsweise Natriumhydrid oder Kaliumhydrid, oder Alkalimetallalkoholate wie Natriummethanolat, Natriumethanolat, Kaliummethanolat, Kaliumethanolat oder Kalium-t.-butylat, oder Carbonate wie Natriumcarbonat, Cäsiumcarbonat oder Kaliumcarbonat oder Amide wie Natriumamid oder Lithiumdiisopropylamid, oder Organolithium-Verbindungen wie Phenyllithium, Butyllithium oder Methyllithium oder Natriumhexamethyldisilazan verwendet werden.

Für die Umsetzung der Verbindungen der Formel (II) zu den Verbindungen der Formel (III) bevorzugte Lösungsmittel sind herkömmliche organische Lösungsmittel, welche sich unter den Reaktionsbedingungen nicht verändern. Vorzugsweise können für das erfindungsgemäße Verfahren Ether wie Diethylether, Butylmethylether, Dioxan, Tetrahydrofuran, Glykoldimethylether oder Diethylenglykoldimethylether, oder Kohlenwasserstoffe wie Benzol, Toluol, Xylol oder Petrolether, oder Alkohole wie Methanol oder Ethanol oder halogenierte Kohlenwasserstoffe wie Tetrachlorkohlenstoff, Chlormethan oder Dichlormethan verwendet werden. Es ist selbstverständlich auch möglich, Gemische der vorstehend genannten Lösungsmittel zu verwenden. Erfindungsgemäß bevorzugt ist die Verwendung von Toluol und/oder Methanol.

Die Verbindungen der Formel (II) werden zunächst mit einem 4-Formylbenzoesäure-C₁₋₆-alkylester zu einer Schiffschen Base umgesetzt und diese dann mit gängigen Reduktionsmitteln, wie z.B. NaBH₄, H₂/Pd/C usw. reduziert oder direkt unter den Bedingungen einer reduktiven Alkylierung in Gegenwart eines Reduktionsmittels, wie z.B. H₂/Pd/C, NaCNBH₃, NaH(OAc)₃ umgesetzt (vgl. Patai, Ed., The Chemistry of the Carbon-Nitrogen Double Bond, S. 276-293 und die dort zitierte Literatur). Je nach Beschaffenheit der Ausgangsverbindung kann hierbei die Umsetzung bei Raumtemperatur erfolgen oder muss auf 50 bis 110°C für mehrere Stunden bis mehrere Tage erhitzt werden. Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt. 4-Formylbenzoesäure-C₁₋₆-alkylester sind kommerziell erhältlich, literaturbekannt, oder können in Analogie zu literaturbekannten Verfahren synthetisiert werden (vgl. z.B. J. Med. Chem. 1989, 32, 1277; Chem. Ber. 1938, 71, 335; Bull. Soc. Chim. Fr. 1996, 123, 679, WO96/11902; DE-2209128; Synthesis 1995, 1135; Bull. Chem. Soc. Jpn. 1985, 58, 2192, Synthesis 1983, 942; J. Am. Chem. Soc. 1992, 114, 8158).

Es sollte vor der Umsetzung der entsprechenden Verbindung der Formel (III) mit dem ω-Halogenvaleriansäurealkylester die vorhandene Methoxygruppe in die freie Hydroxygruppe überführt werden. Dies kann auf bekannte Art erfolgen (vgl. hierzu T.W. Greene, P.G.M. Wuts, Protective Groups in Organic Synthesis, second edition, New York, 1991). Beispielsweise lässt sich die Methylgruppe unter Bildung des Phenols durch Bortribromid in Methylenchlorid bei -70 bis 20°C, durch Trimethylsilyliodid in Chloroform bei 25-50°C oder durch Natriumethylthiolat in DMF bei 150°C abspalten. Erfindungsgemäß ist die Umsetzung mit Bortribromid bevorzugt.

Die Umsetzung der Verbindungen der Formel (III) zu den Verbindungen der Formel (IV) kann vorzugsweise in Acetonitril oder Butyronitril jeweils in Gegenwart einer Base wie Natriumcarbonat, Et₃N, DABCO, K₂CO₃, KOH, NaOH oder NaH durchgeführt werden. Die Reaktion kann im allgemeinen in einem Temperaturbereich von - 20°C bis +90°C, vorzugsweise von 0°C bis +70°C ausgeführt werden. Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt. Als Lösungsmittel kommen jedoch prinzipiell die vorstehend für die Umsetzung der Verbindungen der Formel (II) zu den Verbindungen der Formel (III) genannten Lösungsmittel in Frage. Als ω-Halogenvaleriansäurealkylester wird erfindungsgemäß bevorzugt der entsprechende ω-Bromvaleriansäureethylester verwendet. ω-Halogenvaleriansäurealkylester sind kommerziell erhältlich, literaturbekannt oder können nach literaturbekannten Verfahren synthetisiert werden (vgl. z.B. J. Chem. Soc. 1958, 3065).

Anschließend wird die Umsetzung der Verbindungen der Formel (IV) mit den Verbindungen der Formel (IV-A) durchgeführt. Hierbei handelt es sich um eine nukleophile Substitution einer Abgangsgruppe X in der Verbindung der Formel (IV-A) durch die Hydroxyfunktion der Verbindung der Formel (IV). Als Abgangsgruppe X kommen hierbei beispielsweise in Frage: Halogen, Tosylat, Mesylat, oder eine durch Reagenzien wie Diisopropylazodicarboxylat/PPh₃ aktivierte Hydroxyfunktion (Mitsonobu-Reaktion). Bevorzugt ist X gleich Halogen, besonders bevorzugt gleich Cl. Diese Reaktion kann in einem der vorstehend genannten organischen Lösungsmitteln, vorzugsweise in Acetonitril, durch Reaktion der Verbindungen (IV) und (IV-A) in Gegenwart einer Base wie Natriumcarbonat, NaH, Et₃N, DABCO, K₂CO₃, KOH, NaOH oder vorzugsweise Kaliumcarbonat durchgeführt werden. Die Reaktion kann im allgemeinen in einem Temperaturbereich von 20°C bis +90°C, vorzugsweise von 0°C bis +90°C ausgeführt werden. Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt.

Die Verbindungen der Formel (V) werden anschließend beispielsweise durch Zugabe wässriger Lösungen starker Säuren wie z.B. HCl oder H₂SO₄, oder starker Basen wie z.B. NaOH, KOH oder LiOH in die Verbindungen der Formel (I) durch Hydrolyse der Esterfunktionen zu den freien Carboxylgruppen überführt. Die Reaktion kann in einem der vorstehend genannten organischen Lösungsmitteln, in Wasser oder in Gemischen aus organischen Lösungsmitteln oder in Gemischen aus organischen Lösungsmitteln mit Wasser durchgeführt werden. Erfindungsgemäß bevorzugt ist beispielsweise die Durchführung der Reaktion in einem Gemisch aus Wasser und Methanol oder Dioxan. Die Reaktion kann im allgemeinen in einem Temperaturbereich von -20°C bis +90°C, vorzugsweise von 0°C bis +90°C ausgeführt werden. Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt.

Die Verbindungen der Formel (II) sind kommerziell erhältlich oder literaturbekannt.

Die Verbindungen der Formel (IV-A) sind entweder kommerziell erhältlich, literaturbekannt oder beispielsweise auf folgende Weise zugänglich. Ausgehend von käuflich erhältlichen oder literaturbekannten 2,4-Halogenbenzoesäuren werden zunächst durch Veresterung unter herkömmlichen Bedingungen, beispielsweise durch Chlortrimethylsilan in Methanol, die entsprechenden 2,4-Halogenbenzoesäurealkylester dargestellt, welche durch Umsetzung mit einem Benzolboronsäurederivat in Gegenwart einer Palladiumverbindung sowie gegebenenfalls eines Reduktionsmittels und weiterer Zusatzstoffe im basischen Medium in die entsprechenden Biphenylverbindungen überführt werden. Die letztere Reaktion stellt formal eine reduktive Kupplung dar, wie sie z.B. in L.S. Hegedus, Organometallics in Synthesis; M. Schlosser, Ed., Wiley & Sons, 1994, beschrieben ist. Als Palladiumverbindung kann eine Palladium(II)-Verbindung wie z.B. Cl₂Pd(PPh₃)₂ oder Pd(OAc)₂ oder eine Palladium(0)-Verbindung wie z.B. Pd(PPh₃)₄ oder Pd₂(dba)₃ verwendet werden. Falls erforderlich, können dem Reaktionsgemisch noch zusätzlich ein Reduktionsmittel wie beispielsweise Triphenylphosphin oder andere Zusatzstoffe wie beispielsweise Cu(I)Br, NBu₄NCl, LiCl oder Ag₃PO₄ zugesetzt werden (vgl. hierzu T Jeffery, Tetrahedron lett. 1985, 26, 2667-2670; T. Jeffery, J. Chem. Soc., Chem Commun. 1984, 1287-1289; S. Bräse, A. deMejiere in "Metal-catalyzied cross-coupling reactions", Ed. F. Diederich, P. J. Stang, Wiley-VCH, Weinheim 1998, 99-166). Die Reaktion wird in Gegenwart einer herkömmlichen Base wie z.B. Na₂CO₃, NaOH oder Triethylamin durchgeführt. Als Lösungsmittel kommen die vorstehend genannten organischen Lösungsmittel in Frage, wobei Ether wie beispielsweise 1,2-Dimethoxyethan besonders bevorzugt sind. Die Reaktion kann im allgemeinen in einem Temperaturbereich von -20°C bis +90°C, vorzugsweise von 0°C bis +90°C ausgeführt werden. Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt.

Benzolboronsäuren sind kommerziell erhältlich, literaturbekannt, oder können in Analogie zu literaturbekannten Verfahren synthetisiert werden (vgl. z.B. J.Chem.Soc.C 1966,566. J.Org.Chem., 38,1973,4016).

Anschließend können die Verbindungen der Formel (IV-A) durch Überführung der Carbonsäureesterfunktion durch Reduktion zur entsprechenden Alkoholfunktion mit beispielsweise LiAlH₄ und Überführung der Alkoholfunktion in das entsprechende Halogenid, beispielsweise das entsprechende Chlorid, mit einem herkömmlich verwendeten Halogenierungsmittel wie beispielsweise Thionylchlorid hergestellt werden.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen der allgemeinen Formel (I), führen zu einer Gefäßrelaxation, Thrombozytenaggregationshemmung und zu einer Blutdrucksenkung sowie zu einer Steigerung des koronaren Blutflusses. Diese Wirkungen sind über eine direkte Stimulation der löslichen Guanylatcyclase und einem intrazellulären cGMP-Anstieg vermittelt.

Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorisch und ischämische Attacken, periphere Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose, fibrotischen Erkrankungen wie Leberfibrose oder Lungenfibrose, asthmatischen Erkrankungen und Krankheiten des Urogenitalsystems wie beispielsweise Prostatahypertrophie, erektile Dysfunktion, weibliche sexuelle Dysfunktion und Inkontinenz sowie zur Behandlung von Glaucoma eingesetzt werden.

Die in der vorliegenden Erfindung beschriebenen Verbindungen der allgemeinen Formel (I), stellen auch Wirkstoffe zur Bekämpfung von Krankheiten im Zentralnervensystem dar, die durch Störungen des NO/cGMP-Systems gekennzeichnet sind. Insbesondere sind sie geeignet zur Beseitigung kognitiver Defizite, zur Verbesserung von Lern- und Gedächtnisleistungen und zur Behandlung der Alzheimer'schen Krankheit. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuss- und Suchtmittelaufnahme.

Weiterhin eignen sich die Wirkstoffe auch zur Regulation der cerebralen Durchblutung und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien und des Schädel-Him-Traumas. Ebenso können die erfindungsgemäßen Verbindungen der allgemeinen Formel (I) zur Bekämpfung von Schmerzzuständen eingesetzt werden.

Zudem besitzen die erfindungsgemäßen Verbindungen antiinflammatorische Wirkung und können daher als entzündungshemmende Mittel eingesetzt werden.

Als besonderes und überraschendes Merkmal weisen die Verbindungen der vorliegenden Erfindung eine unerwartet lange Wirkdauer auf.

### Gefäßrelaxierende Wirkung in vitro

Kaninchen werden durch intravenöse Injektion von Thiopental-Natrium narkotisiert bzw. getötet (ca. 50 mg/kg,) und entblutet. Die Arteria Saphena wird entnommen und in 3 mm breite Ringe geteilt. Die Ringe werden einzeln auf je einem triangelförmigen, am Ende offenen Häkchenpaar aus 0,3 mm starkem Spezialdraht (Remanium®) montiert. Jeder Ring wird unter Vorspannung in 5 ml Organbäder mit 37°C warmer, carbogenbegaster Krebs-Henseleit-Lösung folgender Zusammensetzung (mM) gebracht: NaCl: 119; KCl: 4,8; CaCl₂ x 2 H₂O: 1; MgSO₄ x 7 H₂O: 1,4; KH₂PO₄: 1,2; NaHCO₃: 25; Glucose: 10; Rinderserumalbumin: 0,001%. Die Kontraktionskraft wird mit Statham UC2-Zellen erfasst, verstärkt und über A/D-Wandler (DAS-1802 HC, Keithley Instruments München) digitalisiert, sowie parallel auf Linienschreibern registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Testsubstanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50 % zu reduzieren (IC₅₀). Das Standardapplikationsvolumen beträgt 5 µl. Der DMSO-Anteil in der Badlösung entspricht 0,1 %.

Die Ergebnisse sind in Tabelle 1 gezeigt:

**Tabelle 1: Gefäßrelaxierende Wirkung in vitro**

| **Beispiel** | **IC₅₀ (nM)** |
|---|---|
| 2 | 0,15 |
| 6 | 0,17 |
| 9 | 1,8 |
| 12 | 2 |

### Stimulation der rekombinanten löslichen Guanylatcyclase (sGC) in vitro

Die Untersuchungen zur Stimulation der rekombinanten löslichen Guanylatcyclase (sGC) und die erfindungsgemäßen Verbindungen mit und ohne Natriumnitroprussid sowie mit und ohne den Häm-abhängigen sGC-Inhibitor 1*H*-1,2,4-Oxadiazol-(4,3a)-chinoxalin-1-on (ODQ) wurden nach der in folgender Literaturstelle im Detail beschriebenen Methode durchgeführt: M. Hoenicka, E.M. Becker, H. Apeler, T. Sirichoke, H. Schroeder, R. Gerzer und J.-P. Stasch: Purified soluble guanylyl cyclase expressed in a baculovirus/Sf9 system: stimulation by YC-1, nitric oxide, and carbon oxide. J. Mol. Med. 77 (1999): 14-23.

Die Häm-freie Guanylatcyclase wurde durch Zugabe von Tween 20 zum Probenpuffer (0,5 % in der Endkonzentration) erhalten.

Die Aktivierung der sGC durch eine Prüfsubstanz wird als n-fache Stimulation der Basalaktivität angegeben. Die Ergebnisse sind in Tabelle 2 gezeigt:

**Tabelle 2: Stimulation der rekombinanten löslichen Guanylatcyclase (sGC) in vitro**

| Bsp. 6 | Stimulation (n-fach) Häm-haltige sGC | | | | | Häm-freie sGC | |
|---|---|---|---|---|---|---|---|
| Konzentration (µM) | Basal | + DEA/NO (0.001 µM) | + DEA/NO (0.01 µM) | + DEA/NO (0.1 µM) | + ODQ (10 µM) | Basal | + ODQ (10 µM) |
| 0 | 1 | 5 | 30 | 124 | - | 1 | - |
| 0.001 | 2 | 6 | 32 | 127 | 35 | 10 | 25 |
| 0.01 | 9 | 13 | 39 | 134 | 95 | 55 | 61 |
| 0.1 | 18 | 25 | 52 | 157 | 147 | 81 | 79 |
| 1.0 | 23 | 30 | 53 | 151 | 150 | 83 | 83 |
| 10 | 25 | 30 | 67 | 174 | 159 | 84 | 83 |

Aus Tabelle 2 ist ersichtlich, dass eine Stimulation sowohl des Häm-haltigen als auch des Häm-freien Enzyms erreicht wird. Weiterhin zeigt eine Kombination aus sGC-Stimulator und 2-(N,N-Diethylamino)diazenolat-2-oxid (DEA/NO), einem NO-Donor, keine synergistischen Effekt, d.h. die Wirkung von DEA/NO wird nicht potenziert, wie dies bei über einem Häm-abhängigen Mechanismus wirkenden sGC-Stimulatoren zu erwarten wäre. Darüber hinaus wird die Wirkung des erfindungsgemäßen sGC-Stimulators durch den Häm-abhängigen Inhibitor der löslichen Guanylatcyclase ODQ nicht blockiert. Die Ergebnisse aus Tabelle 2 belegen somit den neuen Wirkmechanismus der erfindungsgemäßen Stimulatoren der löslichen Guanylatcyclase.

### Untersuchung der antifibrotischen Substanzwirkung in-vivo

### Methodik

Die antifibrotische Wirkung der Substanzen wurde im Modell der Schweineseruminduzierten Leberfibrose bei der Ratte untersucht. Die Behandlung mit heterologem Serum, z.B. Schweineserum bei Ratten, ist eine häufig in der Literatur angewendete Methode zur Auslösung einer Leberfibrose mit anschließender Zirrhose, die im Gegensatz zu anderen Modellen nur eine minimale Schädigung und Entzündung der Leberzparenchymzellen hervorruft (Bhunchet, E. and Wake, K. (1992): Role of mesenchymal cell populations in porcine serum-induced rat liver fibrosis. Hepatology 16: 1452-1473). Weibliche Sprague Dawley Ratten wurden 2 x wöchentlich mit 0.5 ml/Tier sterilem Schweineserum (Sigma) i.p. behandelt, Kontrolltiere mit steriler physiologischer Kochsalzlösung (2 x wöchentlich 0.5 ml/Tier i.p.).Die Behandlung mit Prüfsubstanz (1 x täglich in 5 ml/kg p.o. Lösungsmittel bestehend aus 20% Cremophor, 10% Transcutol and 70 % H₂O) erfolgte parallel zur Schweineserum-Behandlung. Nach sieben Wochen Behandlung wurden die Tiere getötet und die Lebern zur Quantifizierung des Kollagengehalts entnommen.

Für die histologische Untersuchung des Lebergewebes wurden standardisierte transverse Gewebezylinder (etwa 10 x 2 mm) aus dem rechten Vorderlappen der Leber gestanzt. Gefrierschnitte wurden für die Detektion von durch Leberfibrose hervorgerufenem Narbenkollagen mit 0,1 %iger Pikrosiriusrot-Lösung gefärbt.

Fast Green wurde als Gegenfärbung zur Kontrastverstärkung eingesetzt. Das Ausmaß der Leberfibrose wurde in jedem Schnitt als prozentualer Anteil der Pikrosiriusrotgefärbten Fläche an der gesamten Meßfläche bestimmt. Die Parameter der videomikroskopischen Farbdetektion wurden standardisiert und im gesamten Experiment konstant gehalten. 64 Felder eines standardisierten Rasters von 31 mm² wurden bei 100-facher Endvergrößerung ausgemessen. Für die halbautomatische Morphometrie wurde ein Leica Quantimed 500MC (Leica Deutschland) eingesetzt.
Zur OH-Prolin-Bestimmung nach Prockop und Udenfried (Prockop, D.J. and Udenfried, S.A. (1960): A specific method for the analysis of hydroxyproline in tissues and urine. Anal. Biochem. 1 : 228-239) wurden je 50-100 mg Lebergewebe getrocknet und mit 6N HCl ca. 17 Std gekocht. Nach Verdampfen der Säure im Vakuum-Trockenschrank wurde der Rückstand mit 5 ml Aqua dest. gelöst und filtriert. 200 µl der filtrierten Lösung wurden mit 200 µl Ethanol und 200 µl Oxidationslösung (7%ige wässrige Chloramin T Hydrat-Lösung 1 : 4 mit Acetat-CitratPuffer pH 6.0 verdünnt) 25 min bei Zimmertemperatur inkubiert. Danach wurden 400 µl Ehrlich's Reagenz (12g 4-Dimethylaminobenzaldehyd in 20 ml Ethanol + 2,74 ml konzentrierte Schwefelsäure in 20 ml Ethanol) zugegeben. Nach 3 Stunden Inkubation bei 35°C wurde die Absorption bei 573 nm gemessen. Für die Eichreihe wurden wässrige OH-Prolin (Sigma)-Lösungen verwendet. Der OH-Prolin-Gehalt der Leberproben wurde in mg pro g Leber-Trockengewicht berechnet

### Ergebnisse

Die OH-Prolin-Werte stimmen mit den Ergebnissen der morphometrischen Fibrosemessung sehr gut überein: Die Schweineserum-Behandlung führt ohne gleichzeitige Substanzgabe zu einer ausgeprägten Kollagenakkumulation in der Leber. Die Entstehung dieser Kollagenablagerung wird durch Substanzbehandlung reduziert; mit Beispiel Nr. 6 bereits in den Dosierungen 0,03 mg/kg und 0,1 mg/kg p.o. o.d. signifikant um etwa die Hälfte.

Zur vorliegenden Erfindung gehören pharmazeutische Zubereitungen, die neben nichttoxischen, inerten pharmazeutisch geeigneten Trägerstoffen die erfindungsgemäßen Verbindungen, insbesondere die Verbindungen der allgemeinen Formel (I), enthält sowie Verfahren zur Herstellung dieser Zubereitungen.

Die Wirkstoff können gegebenenfalls in einem oder mehreren der oben angegebenen Trägerstoffe auch in mikroverkapselter Form vorliegen.

Die therapeutisch wirksamen Verbindungen, insbesondere die Verbindungen der allgemeinen Formel (I), sollen in den oben aufgeführten pharmazeutischen Zubereitungen in einer Konzentration von etwa 0,1 bis 99,5, vorzugsweise von etwa 0,5 bis 95 Gew.%, der Gesamtmischung vorhanden sein.

Die oben aufgeführten pharmazeutischen Zubereitungen können außer den erfindungsgemäßen Verbindungen, insbesondere den Verbindungen der allgemeinen Formel (I), auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht

Die vorliegende Erfindung wird nachstehend anhand von nicht einschränkenden bevorzugten Beispielen näher dargestellt. Soweit nicht anderweitig angegeben, beziehen sich alle Mengenangaben auf Gewichtsprozente.

### Beispiele

### Abkürzungen:

- RT:: Raumtemperatur
- EE:: Essigsäureethylester
- BABA:: n-Butylacetat/n-Butanol/Eisessig/Phosphatpuffer pH 6 (50:9:25.15; org. Phase)

### Laufmittel für die Dünnschichtchromatographie:

- T1 E1:: Toluol - Essigsäureethylester (1:1)
- T1 EtOH1:: Toluol-Methanol (1:1)
- C1 E1:: Cyclohexan - Essigsäureethylester (1:1)
- C1 E2:: Cyclohexan - Essigsäureethylester (1:2)

### Ausgangsverbindungen

### Bsp. I: 4-({[2-(2-Methoxyphenyl)ethyl]amino}methyl)benzoesäure-methylester

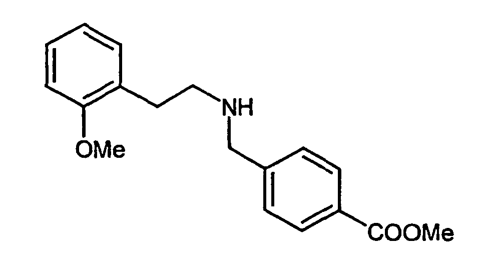

Eine Lösung von 92,08 g (0,597 mol) 2-Methoxyphenethylamin und 98,0 g (0,597 mol) 4-Formylbenzoesäure-methylester in 21 Ethanol wird 2 Stunden zum Rückfluss erhitzt. Anschliessend wird das Lösemittel im Vakuum abgezogen und der erhaltene Rückstand in 11 Methanol gelöst. Portionsweise werden insgesamt 46,14 g fester NaBH₄ zugesetzt. Nach zwei Stunden Rühren bei Raumtemperatur wird der Ansatz in Wasser gegossen und mit Essigester extrahiert. Der organische Extrakt wird mit gesättigter Kochsalz-Lösung gewaschen und über Na₂SO₄ getrocknet. Nach Filtration wird das Lösemittel im Vakuum entfernt. Es werden 167,7 g (0,559 mol, 77% Ausbeute) eines farblosen Öls erhalten, das ohne weitere Reinigung in der nächsten Stufe eingesetzt wird.
¹H-NMR (200 MHz, DMSO-d₆, δ/ppm): 7,90 (2H, d), 7,45 (2H, d), 7,17 (1H, t), 7,12 (1H, d), 6,92 (1H, d), 6,83 (1H, t), 3,83 (3H, s), 3,78 (2H, s), 3,73 (3H, s), 2,75-2,63 (4H, m).
MS (DCI, NH₃): 300 (M+H⁺).

### Bsp. II: 4-({[2-(2-Hydroxyphenyl)ethyl]amino}methyl)benzoesäuremethylester-Hydrobromid

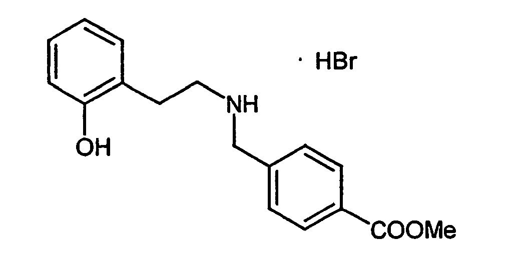

Eine Lösung von 60,0 g (0,2 mol) 4-({[2-(2-Methoxyphenyl)ethyl]amino}methyl)benzoesäure-methylester aus Bsp. I in 200 ml Dichlormethan wird bei 0°C mit 661,4 ml (0,66 mol) einer 1-molaren Lösung von Bortribromid in Dichlormethan versetzt. Man läßt eine Stunde bei 0°C weiterrühren. Dann werden 300 ml Methanol zugefügt und der Ansatz 18 Stunden zum Rückfluss erhitzt. Beim Abkühlen fällt das Produkt aus und wird abfiltriert. Weiteres Produkt wird nach Aufkonzentrieren der Mutterlauge erhalten. Die gesammelten Produktfraktionen werden mit Ether gewaschen. Es werden 45,04 g (0,16 mol, 56% Ausbeute) eines weissen kristallinen Feststoffs erhalten.
R_{f} (Dichlormethan/Methanol 10:1): 0,54.
¹H-NMR (300 MHz, DMSO-d₆, δ/ppm): 9,58 (1H, breit), 9,02 (2H, breit), 8,03 (2H, d), 7,68 (2H, d), 7,09 (1H, d), 7,07 (1H, t), 6,82 (1H, d), 6,77 (1H, t), 4,29 (2H, s), 3,89 (3H, s), 3,18-3,10 (2H, m), 2,94-2,88 (2H, m).
MS (ESI): 286 (M+H⁺).

### Bsp. III: 4-{[[2-(2-Hydroxyphenyl)ethyl](5-methoxy-5-oxopentyl)amino]-methyl}benzoesäure-methylester

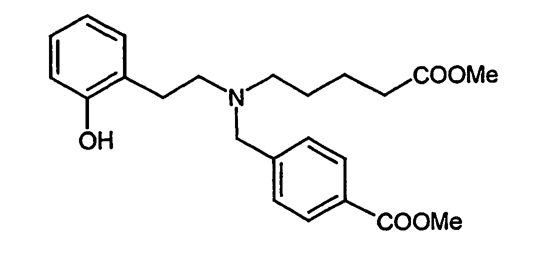

3,0 g (8,19 mmol) 4-({[2-(2-Hydroxyphenyl)ethyl]amino}methyl)benzoesäuremethylester-Hydrobromid aus Bsp. II, 1,3 ml (9,83 mmol) 5-Bromvaleriansäureethylester und 1,74 g (16,38 mmol) wasserfreies Natriumcarbonat werden in 20 ml Acetonitril drei Tage zum Rückfluss erhitzt. Anschliessend wird der Ansatz zur Trockene eingedampft, der Rückstand mit Essigester aufgenommen und mit Wasser und gesättigter Kochsalz-Lösung gewaschen. Nach Trocknen über Na₂SO₄ wird filtriert und eingedampft. Das Produkt wird durch Flash-Chromatographie (Kieselgel, Cyclohexan/Essigester 7:3) gereinigt. Es werden 2,2 g (5,51 mmol, 67% Ausbeute) eines blass gelben Öls erhalten.
R_{f} (Cyclohexan/Essigester 2:1): 0,28.
¹H-NMR (300 MHz, DMSO-d₆, δ/ppm): 9,57 (1H, s breit), 7,89 (2H, d), 7,43 (2H, d), 6,99 (1H, d), 6,98 (1H, t), 6,72 (1H, d), 6,67 (1H, t), 3,83 (3H, s), 3,69 (2H, s), 3,57 (3H, s), 2,71-2,66 (2H, m), 2,62-2,55 (2H, m), 2,45 (2H, t), 2,23 (2H, t), 1,51-1,40 (4H, m).
MS (DCI, NH₃): 400 (M+H⁺), 252.

### Bsp. IV: 4-Brom-2-fluorbenzoesäuremethylester

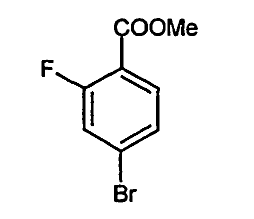

Eine Lösung von 8g (36,53 mmol) 4-Brom-2-fluorbenzoesäure in 44 ml Methanol wird mit 0,46 ml (3,65 mmol) Chlortrimethylsilan versetzt und 12 h zum Rückfluß erhitzt. Anschließend wird der Ansatz eingedampft, mit Cyclohexan aufgenommen und über Kieselgel filtriert. Es werden 4,487 g (19,25 mmol, 52% Ausbeute) eines weißen Feststoff erhalten.
R_{f} (Cyclohexan/Essigester 2:1): 0,5.
¹H-NMR (200 MHz, DMSO-d₆, δ/ppm): 7,84 (1H, t), 7,75 (1H, dd), 7,58 (1H dd), 3,88 (3H, s).
MS (EI): 232 (M⁺).

### Bsp. V: 3-Fluor-4'-methoxy-1,1'-biphenyl-4-carbonsäure-methylester

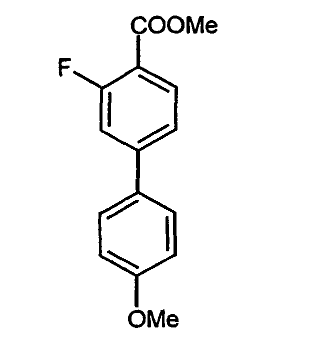

1,45 g (6,22 mmol) 4-Brom-2-fluorbenzoesäuremethylester aus Bsp. IV wird in 15 ml 1,2-Dimethoxyethan gelöst und unter Argon mit 1,13 g (7,47 mmol) 4-Methoxybenzolboronsäure, 80 mg (0,11 mmol) Bis(triphenylphoshin)palladium(II)chlorid und mit 7 ml einer 2-molaren Na₂CO₃-Lösung in Wasser versetzt. Das Reaktionsgemisch wird anschließend 12 h unter Rückfluß gerührt. Nachfolgend wird der Ansatz abgekühlt und über 10g Extrelute filtriert, mit Dichlormethan gewaschen und einrotiert. Das erhaltene Produkt wird durch Flash-Chromatographie (Kieselgel, Cyclohexan/Essigester 2:1) gereinigt. Es werden 1,362g (5,23 mmol, 84% Ausbeute) weißer Feststoff erhalten.
R_{f} (Cyclohexan/Essigester 2:1): 0,44.
¹H-NMR (200 MHz, DMSO-d₆, δ/ppm): 7,96 (1H, t), 7,80-7,62 (2H, m), 7,49-7,28 (3H, m), 7,02 (1H, dd); 3,89 (3H, s), 3,82 (3H, s).
MS (EI): 260 (M⁺).

### Bsp. VI: (3-Fluor-4'-methoxy-1,1'-biphenyl-4-yl)-methanol

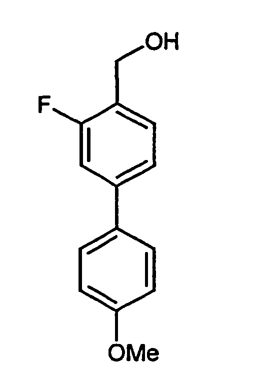

3,14 ml (3,14 mmol) einer 1-molaren Lösung von LiAlH₄ wasserfreiem THF werden bei 0°C tropfenweise mit einer Lösung von 1,36 g (5,23 mmol) 3-Fluor-4'-methoxy-1,1'-biphenyl-4-carbonsäure-methylester aus bsp. V in 10 ml wasserfreiem THF versetzt. Es wird zunächst 2 h bei 0°C gerührt. Anschliessend wird vorsichtig mit 10 ml ges. NH₄Cl-Lösung versetzt, mit Essigester verdünnt und die organische Phase abgetrennt. Die organische Phase wird nacheinander mit Wasser und gesättigter Kochsalz-Lösung gewaschen, über wasserfreiem Natriumsulfat getrocknet und nach Filtration vom Lösemittel befreit. Das erhaltene Rohprodukt wird durch Flash-Chromatographie (Kieselgel, Cyclohexan/Essigester 5:1) gereinigt. Es werden 914 mg (3,94 mmol, 73% Ausbeute) (3-Fluor-4'-methoxy-1,1'-biphenyl-4-yl)-methanol erhalten.
R_{f}(Cyclohexan/Essigester 2:1): 0,29.
¹H-NMR (300 MHz, DMSO-d₆, δ/ppm): 7,58-7,45 (3H, m), 7,39 (1H, t), 7,29-7,20 (2H, m), 6,95 (1H, dd), 5,30 (1H, t), 4,58 (2H, d), 3,83 (3H, s).
MS (EI): 232 (M⁺).

### Bsp. VII: 4-(Chlormethyl)-3-fluor-4'-methoxy-1,1'-biphenyl

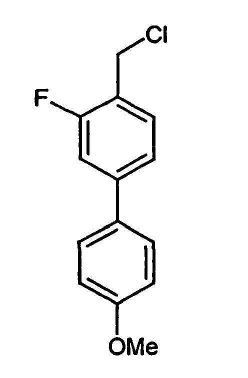

Eine Lösung von 864 mg (3,72 mmol) (3-Fluor-4'-methoxy-1,1'-biphenyl-4-yl)-methanol aus Bsp. VI in 3 ml Chloroform wird langsam mit 0,54 ml (7,45 mmol) Thionylchlorid, gelöst in 2ml Chloroform, versetzt und 12 h bei Raumtemperatur gerührt. Anschließend wird der Ansatz eingedampft, mit Essigester aufgenommen und mit Wasser und zweimal mit ges. NaHCO₃-Lösung gewaschen. Nach Trocknen über MgSO₄, Filtration und Einengen wird das Produkt durch Flash-Chromatographie (Kieselgel Cyclohexan/Essigester 100:1) gereinigt. Es werden 511 mg (2,04 mmol, 55% Ausbeute) eines farblosen Öls erhalten.
R_{f} (Cyclohexan/Essigester 2:1): 0,52.
¹H-NMR (200 MHz, DMSO-d₆, δ/ppm): 7,68-7,52 (3H, m), 7,46-7,21 (3H, m), 6,99 (1H, dd), 4,86 (2H, s), 3,86 (3H, s).
MS (EI): 250 (M⁺).

### Bsp. VIII: 4-{[(2-{2-[(3-Fluor-4'-methoxy-1,1'-biphenyl-4-yl)-methoxy]-phenyl}ethyl)(5-ethoxy-5-oxopentyl)-amino]-methyl}-benzoesäure-methylester

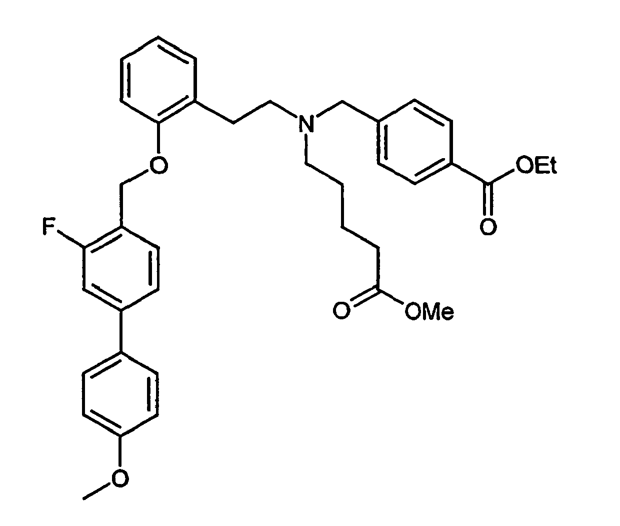

Eine Lösung von 511 mg (2,04 mmol) 4-(Chlormethyl)-3-fluor-4'-methoxy-1,1'-biphenyl aus Bsp. VII in 7 ml trockenem Acetonitril wird mit 702 mg (1,7 mmol) 4-{[[2-(2-Hydroxyphenyl)ethyl](5-ethoxy-5-oxopentyl)amino]methyl}bcnzoesäuremethylester aus Bsp. III und 352 mg (2,55 mol) wasserfreiem Kaliumcarbonat versetzt und 12 Stunden zum Rückfluß erhitzt. Anschließend wird der Ansatz zur Trockene einrotiert. Das erhaltene Rohprodukt wird durch Flash-Chromatographie (Cyclohexan/Essigester 6:1) gereinigt. Es werden 1,018 g (1,62 mmol, 94 % Ausbeute) eines Feststoffs erhalten.
R_{f} (Cyclohexan/Essigester 2:1): 0,30.
¹H-NMR (300 MHz, DMSO-d₆, δ/ppm): 7,80 (2H, d), 7,56 (1H, d) 7,51-7,48 (2H, m), 7,41-7,05 (8H, m), 6,98 (1H, dd), 6,89 (1H, t), 5,12 (2H, s), 3,99 (2H, q), 3,86 (3H, s), 3,81 (3H, s), 3,59 (2H, s), 2,78-2,68 (2H, m), 2,63-2,53 (2H, m), 2,39 (2H, t), 2,11 (2H, t), 1,48-1,27 (4H, m), 1,10 (3H, t).
MS (ESI): 628 (M+H⁺).

### Synthesebeispiele

### Bsp. 1: 4-{[(4-Carboxybutyl)(2-{2-[(3-fluor-4'-methoxy-1,1'-biphenyl-4-yl)methoxy]phenyl}ethyl)amino]methyl}benzoesäure

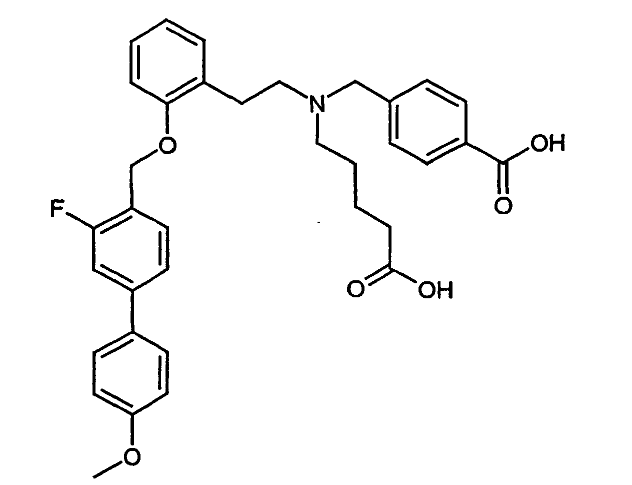

Eine Lösung von 222 mg (0,37 mmol) 4-{[(2-{2-[(3-Fluor-4'-methoxy-1,1'-biphenyl-4-yl)-methoxy]-phenyl}-ethyl)(5-ethoxy-5-oxopentyl)-amino]-methyl}-benzoesäuremethylester aus Bsp. VIII in 10 ml Dioxan und 5 ml Wasser wird mit 0,46 ml einer 45 %igen Lösung von NaOH in Wasser versetzt und 2 Stunden lang bei 90°C gerührt. Nach dem Abkühlen wird das Dioxan abgezogen und die wässrige Phase wird mit 1-molarer Salzsäure auf pH 4 bis 5 eingestellt. Dabei fällt das Produkt aus, das abfiltriert, mit Wasser gewaschen und getrocknet wird. Es werden 738 mg (1,26 mmol, 81 % Ausbeute) eines weißen Feststoffs erhalten.
R_{f} (Dichlormethan/Methanol 7:3): 0,25.
¹H-NMR (200 MHz, DMSO-d₆, δ/ppm): 12,99-12,0 (2H, breites s), 7,82 (2H, d), 7,63 (2H, d) 7,54-7,41 (3H, m), 7,31 (2H, d), 7,24-7,03 (3H, m), 7,01 (2H, d), 6,89 (1H, t), 5,09 (2H, s), 3,80 (3H, s), 3,59 (2H, breites s), 2,78-2,65 (2H, m), 2,63-2,53 (2H, m), 2,45-2,31 (2H, m), 2,09 (2H, t), 1,47-1,30 (4H, m).
MS (ESI): 586 (M+H⁺).

Auf analoge Weise wurde erhalten:

| Bsp. | Formel | ¹H-NMR δ [ppm] (DMSO-d₆) |
|---|---|---|
| 2 (ausgehend von 4-t-Butylbenzolboronsäure) | 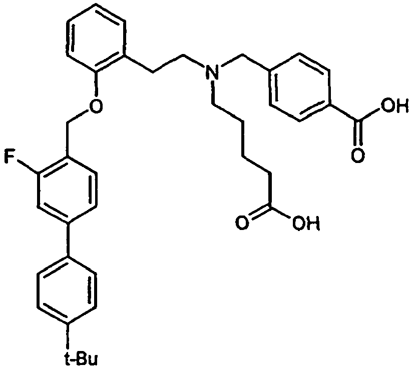 | 12,32(2H,breit),7,84(2H,d), 7,61 (2H, d), 7,58-7,44 (5H, m), 7,41-7,28 (2H, m), 7,25-7,05 (3H, m), 6,89 (1H, t), 5,12 (2H ,s), 3,80-3,51 (2H, m), 2,81-2,57 (4H, m), 2,55-2,38 (2H+DMSO, m), 2,10 (2H, t), 1,49-1,38 (4H, m), 1,31 (9H, s). (300 MHz) |
| 3 (ausgehend von 4-Trifluormethylbenzolboronsäure) | 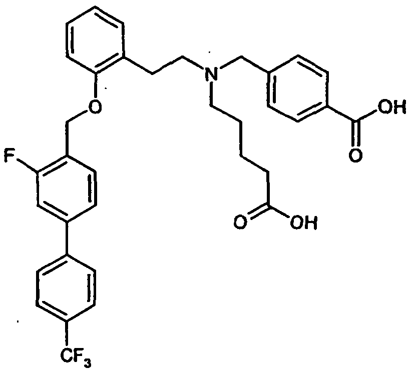 | 12,5 (2H, breit), 8,01-7,87 (4H, m), 7,80 (2H, d), 7,73-7,54 (5H, m), 7,32-7,09 (3H, m), 6,94 (1H, t), 5,20 (2H ,s), 4,48-4,30 (2H, m), 3,20-2,90 (4H, m),2,52-2,42 (2H+DMSO, m), 2,14 (2H, t), 1,75-1,57 (2H, m), 1,48-1,32 (2H, in). (300 MHz) |
| 4 (ausgehend von 4-Brom-2-chlorbenzoesäure und 4-Methoxybenzolboronsäure) | 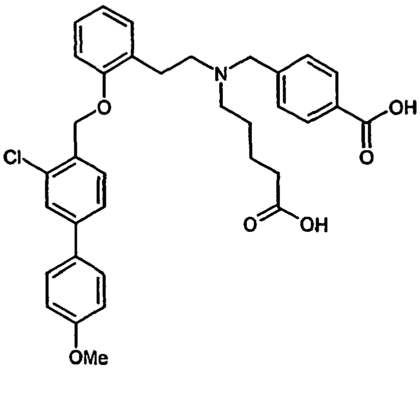 | 12,45 (2H, breites s), 7,89-7,77 (2H, m), 7,74 (1H, s), 7,63 (2H, d), 7,59 (2H, s), 7,42-7,29 (2H, m), 7,17 (2H, t), 7,10-6,97 (3H, m), 6,89 (1H, t), 5,12 (2H ,s), 3,82 (3H, s), 3,70-3,51 (2H, m), 2,91-2,32 (6H+DMSO, m), 2,09 (2H, t), 1,51-1,39 (4H, m). (200 MHz) |
| 5 (ausgehend von 4-Brom-2-chlorbenzoesäure und 4-Fluorbenzolboronsäure) | 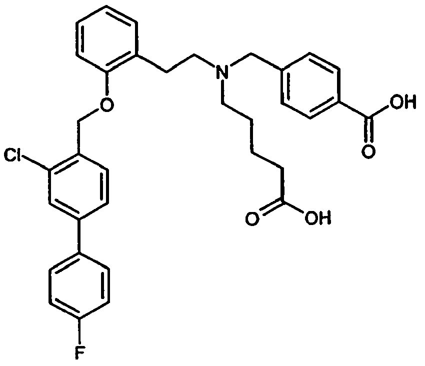 | 12,7-12,1 (2H, breit), 7,91-7,89 (2H, m), 7,88 (1H, d), 7,58 (2H, dd), 7,47 (2H, d), 7,42-6,99 (6H,m), 7,02 (1H, d), 6,89 (1H, t), 5,07 (2H,s), 3,68-3,49(2H,breit), 2,90-2,30 (6H+DMSO, m), 2,12 (2H, m), 1,52-1,30 (4H, m). (200 MHz) |
| 6 (ausgehend von 4-Brom-2-chlorbenzoesäure und 4-Trifluormethylbenzolboronsäure) | 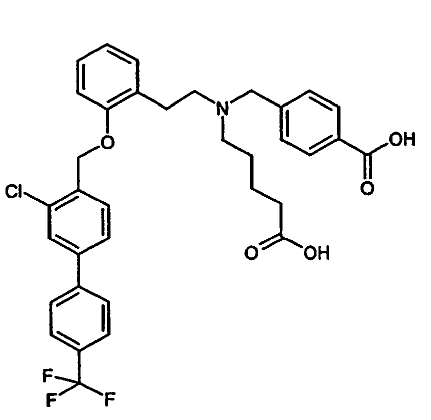 | 12,40 (2H, breit), 8,01-7,52 (11H, m), 7,38-7,03 (3H, m), 7,02-6,85 (1H, m), 5,20 (2H, s), 4,39 (2H, s), 3,30-2,89 (6H, m), 2,21-2,02 (2H, m), 1,74-1,52 (2H, m), 1,48-1,28 (2H, m). (300 MHz) |
| 7 (ausgehend von 4-Chlorbenzolboronsäure) | 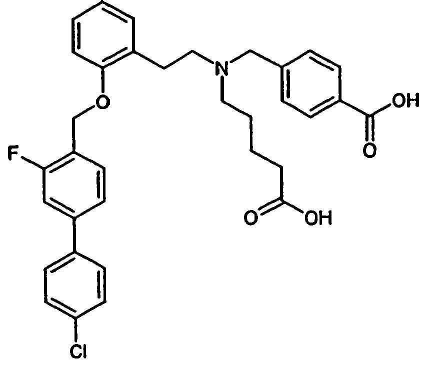 | 12,2 (2H, breit), 7,99-7,78 (2H, m), 7,71 (2H, d), 7,62-7,43 (6H, m), 7,40-7,01 (4H, m), 6,90 (1H, t), 5,14 (2H, s), von 4,60-1,28 (14H, m) darin 2,18-2,01 (2H, m), 1,49-1,28 (2H, m). (300 MHz) |
| 8 (ausgehend von 1-Cyclohex-1-enboronsäure) | 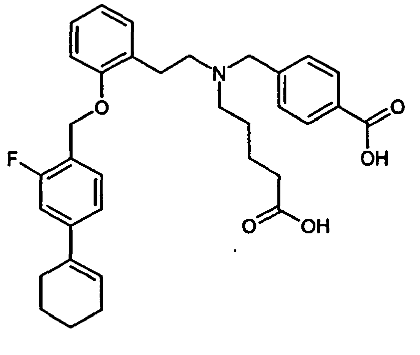 | 12,4 (2H, breit), 7,81 (2H, d), 7,44-6,99 (8H, m), 6,85 (1H, t), 6,24 (1H, t), 5,03 (2H, s),3,57 (2H, s), 2,78-2,62 (2H, m), 2,61-2,45 (2H+DMSO, m), 2,44-2,26 (4H, m), 2,25-2,00 (4H, m), 1,79-1,50 (4H, m), 1,48-1,28 (4H, m). (200 MHz) |
| 9 (ausgehend von 4-Brom-2-chlorbenzoesäure und 1-Cyclohex-1-enboronsäure) | 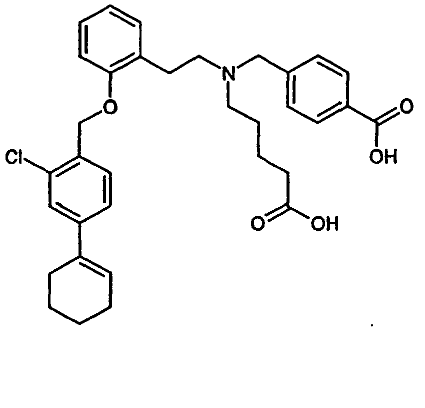 | 12,3 (1H, breit), 10,2 (1H, breit), 7,98(2H,d), 7,72-7,57 (2H,m), 7,54-7,42 (2H, m), 7,36 (1H, d), 7,31-7,17(2H, m), 7,10 (1H,d), 6,95(1H,t), 6,24 (1H,t), 5,12(2H,s), 4,38 (2H, s), 3,32-2,89 (5H, m), 2,39-2,26(2H,m), 2,25-2,04 (5H, m), 1,81-1,48 (6H, m), 1,47-1,27(2H,m).(200 MHz) |
| 10 (ausgehend von 4-Brom-2-chlorbenzoesäure und 4-t-Butylbenzolboronsäure) | 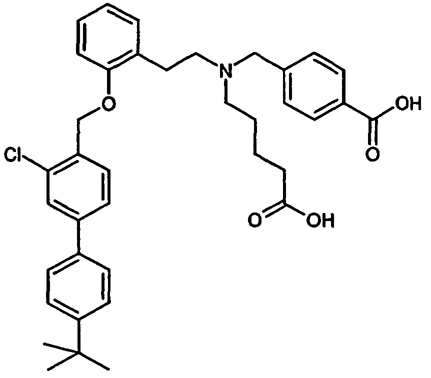 | 12,4 (2H, breit), 7,89-7,78 (2H, m), 7,77 (1H, s) 7,64-7,53 (4H, m), 7,49 (2H, d), 7,44-7,28 (2H, m), 7,27-7,11 (2H, m), 7,08 (1H, d), 6,91 (1H, t), 5,13 (2H, s), 3,75-3,52 (2H, breit), 2,90-2,60 (6H, breit), 2,11 (2H, t), 1,49-1,38 (4H, m), 1,33 (9H, s). (300 MHz) |
| 11 (ausgehend von 1-Cyclohexanboronsäure) | 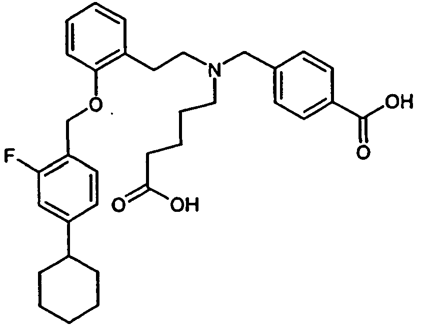 | 12,4 (2H, breit), 7,99-7,74 (2H, breit), 7,43-6,80 (9H, m), 5,02 (2H, s), 3,58 (2H, breit), 3,18-2,28 (5H + DMSO, m), 2,11 (2H, t), 1,87-1,60 (6H, m), 1,52-1,18 (10H, m). (300 MHz) |
| 12 (ausgehend von 4-Brom-2-chlorbenzoesäure und 1-Cyclohexanboronsäure) | 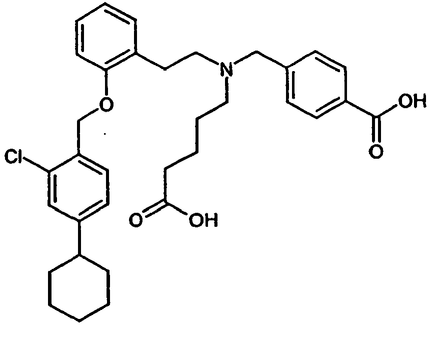 | 12,4 (2H, breit), 7,99-7,73 (2H, breit), 7,42-6,80 (2H, d), 7,32 (1H, pseudo-s), 7,18 (4H, dd), 7,06 (1H,d), 6,90 (1H, t), 5,07 (2H, s), 3,58 (2H, breit), 3,20-2,28 (5H+DMSO, m), 2,11 (2H, t), 1,88-1,61 (6H, m), 1,52-1,10 (10H, m). (200 MHz) |

### Bsp. 13: 4-{[(4-Carboxybutyl)(2-{2-[(3-fluor-4-trifluormethyl-1,1'-biphenyl-4-yl)methoxy]phenyl}ethyl)amiao]methyl}benzoesäure-Hydrochlorid

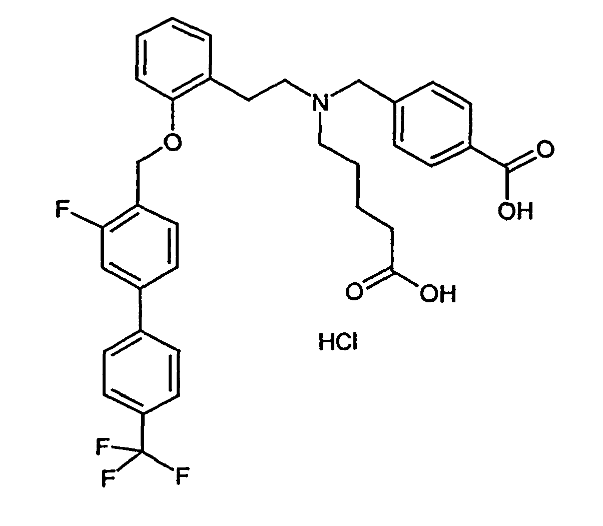

Eine Lösung von 220 mg 4-{[(4-Carboxybutyl)(2-{2-[(3-fluor-4'-trifluormethyl-1,1'-biphenyl-4-yl)methoxy]phenyl}ethyl)amino]methyl}benzoesäure aus Bsp. 3 in 0,2 ml Dioxan werden mit 0,5 ml (2 mmol) einer 4-molaren Lösung von HCl in Dioxan versetzt und 1 h bei 60°C gerührt. Anschließend wird der Ansatz eingedampft und das erhaltene farblose Öl mehrmals mit Diethylether verrührt. Die entstandenen Kristalle werden filtriert und getrocknet.
¹H-NMR: δ [ppm] (DMSO-d₆): 12,60 (2H, breit), 10,40 (1H, breit), 8,02-7,78 (6H, m), 7,76-7,54 (5H, m), 7,37-7,08 (3H, m), 6,93 (1H, t), 5,19 (2H, s), 4,40 (2H, s), 3,22-2,89 (6H, m), 2,14 (2H, t), 1,79-1,52 (2H, m), 1,50-1,29 (2H, m). (200 MHz) MS (ESI): 624 (M+H-HCl+).

## Patentansprüche

1. Verbindungen der allgemeinen Formel (I) 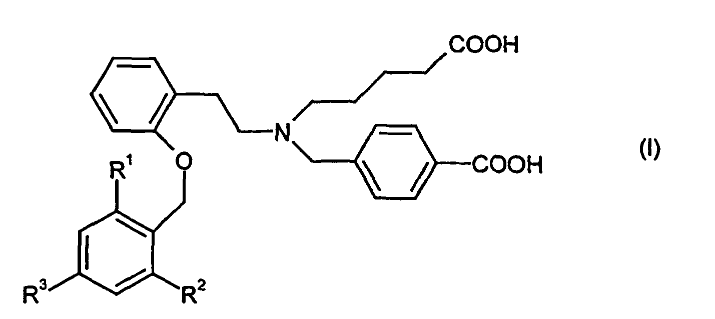 worin
R¹ Halogen bedeutet;
R² H oder Halogen bedeutet;
R³ C₃₋₈-Cycloalkyl, C₃₋₈-Cycloalkenyl oder Phenyl bedeutet, wobei der Phenylrest zusätzlich einen Substituenten aus der Gruppe, bestehend aus Halogen, C₁₋₆-Alkyl, C₁₋₆-Alkoxy, CF₃, tragen kann;
sowie Salze, Isomere und Hydrate davon.

2. Verbindungen nach Anspruch 1, **dadurch, gekennzeichnet, dass**
R¹ F oder Cl bedeutet;
R² H bedeutet;
R³ C₃₋₈-Cycloalkyl, C₃₋₈-Cycloalkenyl oder Phenyl bedeutet, wobei der Phenylrest zusätzlich einen Substituenten aus der Gruppe, bestehend aus F, Cl, Br, Methyl, Ethyl, n-Propyl, i-Propyl, n-Butyl, i-Butyl, t-Butyl, Methoxy, Ethoxy, n-Propoxy, i-Propoxy, n-Butyloxy, i-Butyloxy, t-Butyloxy" CF₃, tragen kann;
sowie Salze, Isomere und Hydrate davon.

3. Verbindungen nach Anspruch 1, **dadurch, gekennzeichnet, dass**
R¹ F oder Cl bedeutet;
R² H bedeutet;
R³ Cyclohexyl, 1-Cyclohexenyl oder Phenyl bedeutet, wobei der Phenylrest zusätzlich einen Substituenten aus der Gruppe, bestehend aus F, Cl, t-Butyl, OMe, CF₃, tragen kann;
sowie Salze, Isomere und Hydrate davon.

4. Verfahren zur Herstellung von Verbindungen der allgemeinen Formel (I), **dadurch gekennzeichnet, dass** man Verbindungen der Formel (II) 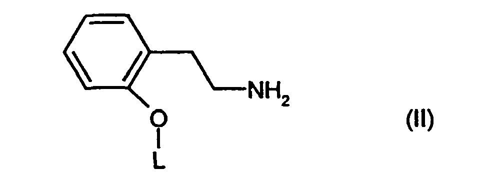 worin
L für Methyl steht,
mit einem 4-Formylbenzoesäure-C₁₋₆-alkylester in einem organischen Lösungsmittel gegebenenfalls unter Erhitzen und gleichzeitiger oder anschließender Zugabe eines Reduktionsmittels zu Verbindungen der Formel (III) umsetzt, 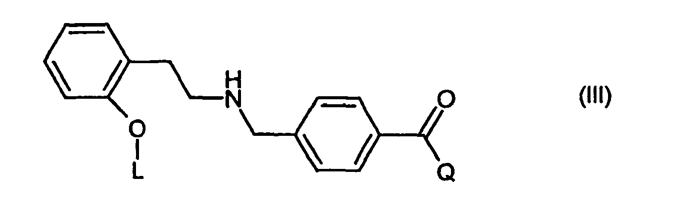 worin
L die vorstehend angegebene Bedeutung hat und Q für einen C₁₋₆-Alkylrest steht,
anschließend - unter vorheriger Etherspaltung zur freien Hydroxylgruppe - mit einem ω-Halogenvaleriansäure-C₁₋₆-alkylester in einem organischen Lösungsmittel in Gegenwart einer Base unter Erhitzen zu Verbindungen der Formel (IV) umsetzt, 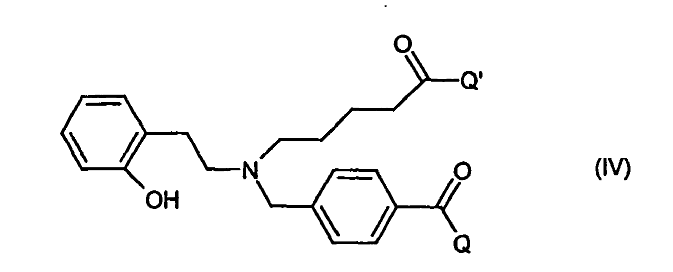 worin
Q die vorstehend angegebene Bedeutung hat und Q' für einen C₁₋₆-Alkylrest steht,
anschließend mit einer Verbindung der Formel IV-A in einem organischen Lösungsmittel in Gegenwart einer Base unter Erhitzen 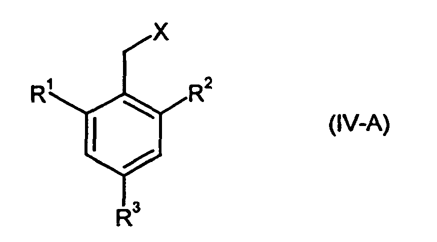 wobei
R¹, R² und R³ die vorstehend angegebenen Bedeutung haben und X für Halogen steht,
zu Verbindungen der Formel (V) umsetzt, 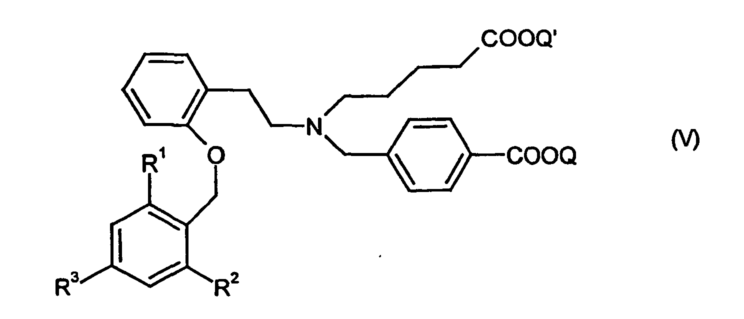 und anschließend die Verbindungen der Formel (V) unter alkalischen Bedingungen zu den Verbindungen der Formel (I) hydrolysiert.

5. Verbindungen nach einem der Ansprüche 1 bis 3 zur Behandlung von Krankheiten.

6. Arzneimittel enthaltend mindestens eine Verbindung der allgemeinen Formel (I) gemäß einem der vorhergehenden Ansprüche.

7. Verwendung von Verbindungen der Formel (I) gemäß einem der vorhergehenden Ansprüche zur Herstellung eines Arzneimittels zur Behandlung von Herz-Kreislauf-Erkrankungen.

8. Verwendung von Verbindungen der allgemeinen Formel (I) gemäß einem der vorhergehenden Ansprüche zur Herstellung von Arzneimitteln zur Behandlung von Angina pectoris, Ischämien und Herzinsuffizienz.

9. Verwendung von Verbindungen der allgemeinen Formel (I) gemäß einem der vorhergehenden Ansprüche zur Herstellung von Arzneimitteln zur Behandlung von Hypertonie, thromboembolischen Erkrankungen, Arteriosklerose und venösen Erkrankungen.

10. Verwendung von Verbindungen der allgemeinen Formel (I) gemäß einem der vorhergehenden Ansprüche zur Herstellung von Arzneimitteln zur Behandlung von fibrotischen Erkrankungen.

11. Verwendung nach Anspruch 10, **dadurch gekennzeichnet, dass** die fibrotische Erkrankung Leberfibrose ist.

## Claims

1. Compounds of the general formula (I) 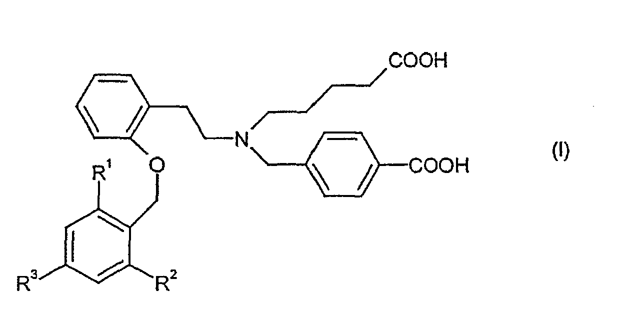 where
R¹ represents halogen;
R² represents H or halogen;
R³ represents C₃₋₈-cycloalkyl, C₃₋₈-cycloalkenyl or phenyl, where the phenyl radical may additionally carry a substituent from the group consisting of halogen, C₁₋₆-alkyl, C₁₋₆-alkoxy, CF₃;
and salts, isomers and hydrates thereof.

2. Compounds according to Claim 1, **characterized in that**
R¹ represents F or Cl;
R² represents H;
R³ represents C₃₋₈-cycloalkyl, C₃₋₈-cycloalkenyl or phenyl, where the phenyl radical may additionally carry a substituent from the group consisting of F, Cl, Br, methyl, ethyl, n-propyl, i-propyl, n-butyl, i-butyl, t-butyl, methoxy, ethoxy, n-propoxy, i-propoxy, n-butyloxy, i-butyloxy, t-butyloxy, CF₃;
and salts, isomers and hydrates thereof.

3. Compounds according to Claim 1, **characterized in that**
R¹ represents F or Cl;
R² represents H;
R³ represents cyclohexyl, 1-cyclohexenyl or phenyl, where the phenyl radical may additionally carry a substituent from the group consisting of F, Cl, t-butyl, OMe, CF₃;
and salts, isomers and hydrates thereof.

4. Process for preparing compounds of the general formula (I), **characterized in that** compounds of the formula (II) 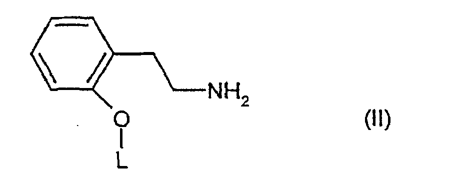 where
L represents methyl
are reacted with a C₁₋₆-alkyl 4-formylbenzoate in an organic solvent, if appropriate with heating and simultaneous or subsequent addition of a reducing agent, to give compounds of the formula (III) 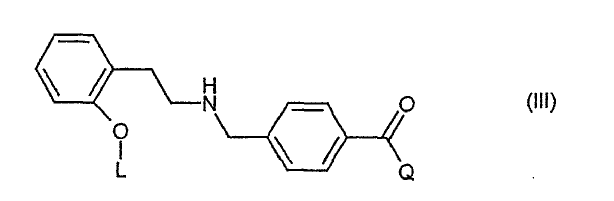 where
L is as defined above and Q represents a C₁₋₆-alkyl radical,
subsequently - with prior ether cleavage to the free hydroxyl group - reacted with a C₁₋₆-alkyl ω-halovalerate in an organic solvent in the presence of a base with heating to give compounds of the formula (IV) 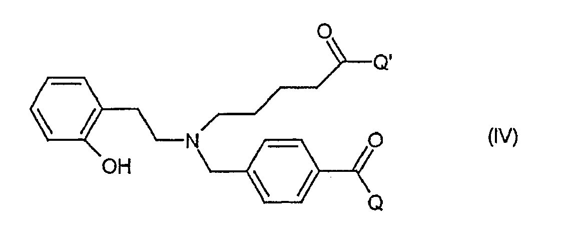 where
Q is as defined above and Q' represents a C₁₋₆-alkyl radical,
then reacted with a compound of the formula IV-A in an organic solvent in the presence of a base with heating 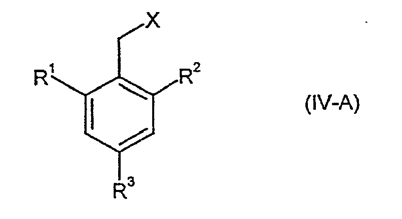 where
R¹, R² and R³ are as defined above and X represents halogen
to give compounds of the formula (V) 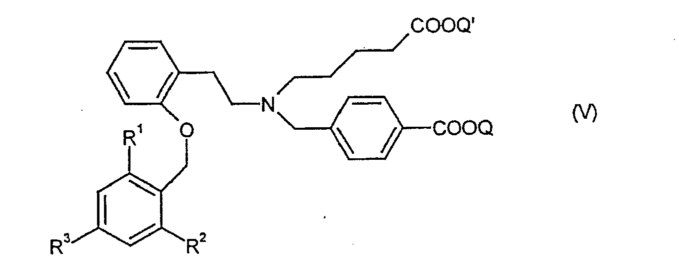 and the compounds of the formula (V) are then hydrolyzed under alkaline conditions to give the compounds of the formula (I).

5. Compounds according to any of Claims 1 to 3 for treating diseases.

6. Medicament, comprising at least one compound of the general formula (I) according to any of the preceding claims.

7. Use of compounds of the formula (I) according to any of the preceding claims for preparing a medicament for treating cardiovascular disorders.

8. Use of compounds of the general formula (I) according to any of the preceding claims for preparing medicaments for treating angina pectoris, ischemias and heart failure.

9. Use of compounds of the general formula (I) according to any of the preceding claims for preparing medicaments for treating hypertension, thromboembolic disorders, arteriosclerosis and venous diseases.

10. Use of compounds of the general formula (I) according to any of the preceding claims for preparing medicaments for treating fibrotic disorders.

11. Use according to Claim 10, **characterized in that** the fibrotic disorder is fibrosis of the liver.

## Revendications

1. Composés de formule générale (I) : 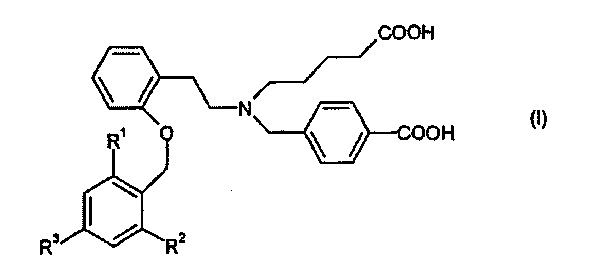 dans laquelle :
R¹ représente un halogène ;
R² représente H ou un halogène ;
R₃ est un reste cycloalkyle en C₃ à C₈, cycloalcényle en C₃ à C₈ ou phényle, le reste phényle pouvant en outre porter un substituant du groupe comprenant un halogène, un radical alkyle en C₁ à C₆, alkoxy en C₁ à C₆, CF₃
ainsi que leurs sels, leurs isomères et leurs hydrates.

2. Composés suivant la revendication 1, **caractérisés en ce que** :
R¹ représente F ou Cl ;
R² représente H ;
R³ est un reste cycloalkyle en C₃ à C₈, cycloalcényle en C₃ à C₈ ou phényle, le reste phényle pouvant en outre porter un substituant du groupe constitué des radicaux F, Cl, Br, méthyle, éthyle, n-propyle, isopropyle, n-butyle, isobutyle, tertiobutyle, méthoxy, éthoxy, n-propoxy, isopropoxy, n-butyloxy, isobutyloxy, tertiobutyloxy, CF₃ ;
ainsi que leurs sels, leurs isomères et leurs hydrates.

3. Composés suivant la revendication 1, **caractérisés en ce que** :
R¹ représente F ou Cl ;
R² représente H ;
R³ est un reste cyclohexyle, 1-cyclohexényle ou phényle, le reste phényle pouvant en outre porter un substituant du groupe comprenant les radicaux F, Cl, tertiobutyle, OMe, CF₃ ;
ainsi que leurs sels, leurs isomères et leurs hydrates.

4. Procédé de production de composés de formule générale (I), **caractérisé en ce qu'**on fait réagir des composés de formule (II) : 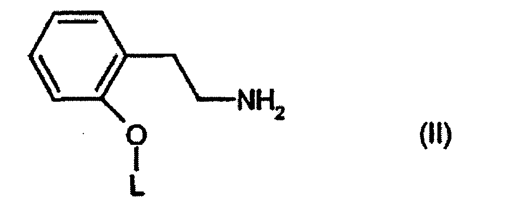 dans laquelle
L est un reste méthyle,
avec un ester d'alkyle en C₁ à C₆ de l'acide 4-formylbenzoïque dans un solvant organique, éventuellement à chaud et avec addition simultanée ou subséquente d'un agent réducteur pour former des composés de formule (III) : 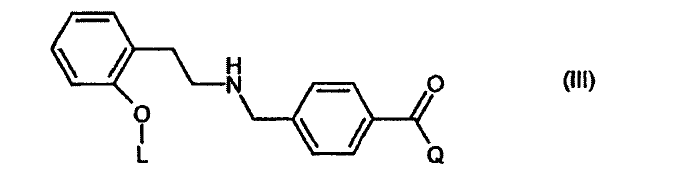 dans laquelle :
L a la définition indiquée ci-dessus et Q représente un reste alkyle en C₁ à C₆,
qu'on fait ensuite réagir en chauffant - avec clivage préalable de l'éther en groupe hydroxyle libre - avec un ester d'alkyle en C₁ à C₆ d'acide ω-halogénovalérique dans un solvant organique en présence d'une base pour obtenir des composés de formule (IV) : 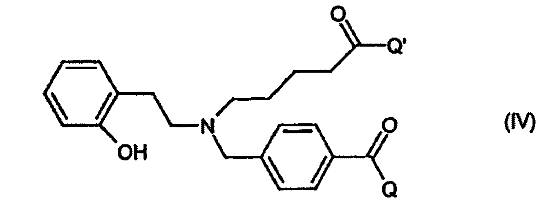 dans laquelle :
Q a la définition indiquée ci-dessus et Q' représente un reste alkyle en C₁ à C₆,
puis, en chauffant, avec un composé de formule IV-A dans un solvant organique en présence d'une base 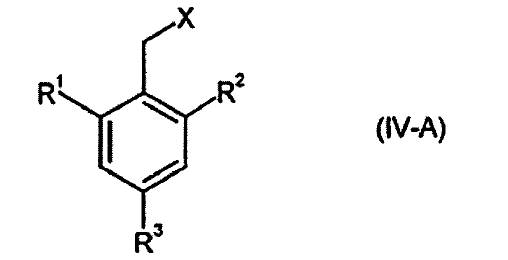 formule dans laquelle :
R¹, R² et R³ ont la définition indiquée ci-dessus et X représente un halogène,
pour obtenir des composés de formule (V) : 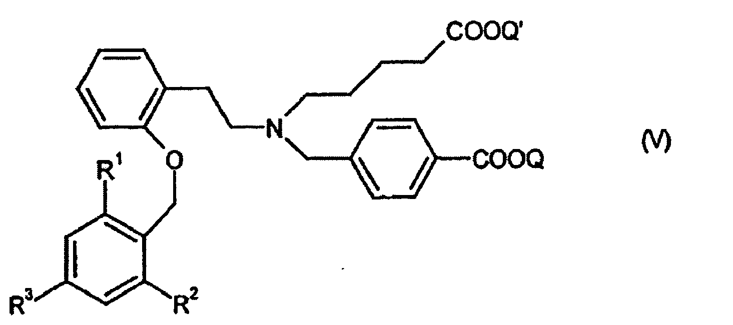 après quoi les composés de formule (V) sont hydrolysés dans des conditions alcalines en composés de formule (I).

5. Composés suivant l'une des revendications 1 à 3, destinés au traitement de maladies.

6. Médicament contenant au moins un composé de formule générale (I) suivant l'une des revendications précédentes.

7. Utilisation de composés de formule (I) suivant l'une des revendications précédentes, pour la préparation d'un médicament destiné au traitement de maladies cardio-vasculaires.

8. Utilisation de composés de formule générale (I) suivant l'une des revendications précédentes, pour la préparation de médicaments destinés au traitement de l'angine de poitrine, d'ischémies et de l'insuffisance cardiaque.

9. Utilisation de composés de formule générale (I) suivant l'une des revendications précédentes, pour la préparation de médicaments destinés au traitement de l'hypertonie, de maladies thromboemboliques, de l'artériosclérose et d'affections veineuses.

10. Utilisation de composés de formule générale (I) suivant l'une des revendications précédentes, pour la préparation de médicaments destinés au traitement de fibroses.

11. Utilisation suivant la revendication 9, **caractérisée en ce que** la fibrose est une fibrose hépatique.
